Europäisches Patentamt

European Patent Office

Office européen des brevets

(19)

(11) Publication number: **0 239 361 B1**

## (12) EUROPEAN PATENT SPECIFICATION

(45) Date of publication of patent specification: **02.12.92**  (51) Int. Cl.⁵: **A61K 9/54**

(21) Application number: **87302518.3**

(22) Date of filing: **24.03.87**

(54) Sustained-release pharmaceutical preparation.

(30) Priority: **27.03.86 US 844676**
**24.02.87 US 17988**

(43) Date of publication of application:
**30.09.87 Bulletin  87/40**

(45) Publication of the grant of the patent:
**02.12.92 Bulletin  92/49**

(84) Designated Contracting States:
**BE CH DE FR GB IT LI NL SE**

(56) References cited:
**EP-A- 0 130 162**
**EP-A- 0 148 811**
**FR-A- 2 184 902**
**FR-A- 2 287 216**
**GB-A- 2 066 070**

(73) Proprietor: **KINAFORM TECHNOLOGY, INC.**
**1101-C Lyons Road**
**Dayton Ohio 45459(US)**

(72) Inventor: **Eichel, Herman J.**
**2571 Clarion Court**
**Columbus, OH 43220(US)**
Inventor: **Massmann, Brent D.**
**684 Riverview**
**Columbus, OH 43202(US)**

(74) Representative: **Bankes, Stephen Charles Dig-by et al**
**BARON & WARREN 18 South End Kensington**
**London W8 5BU(GB)**

## Description

The present invention relates to sustained-release pharmaceutical preparations and the method for making them. More particularly, it relates to a granular, water-soluble drug, such as aspirin, coated with a dual wall structure to give a delayed and sustained drug delivery. The dual walled coated drug may be mixed with uncoated drug and/or single walled coated drug to provide an improved sustained-release system.

As is well known, the maximum time of effectiveness in many pharmaceutical preparations, particularly those containing a water soluble drug such as aspirin, is only a few hours because of biological modification and/or elimination of the medication in the body. Consequently, repeated dosages must be taken at frequent intervals to obtain long term pain relief. Furthermore, aspirin usually dissolves readily in the gastric juices of the stomach and the total dosage is immediately fed into the blood stream. The level of aspirin in the blood stream constantly decreases because of the biological elimination, so there is little or no pain relief at the end of the period between dosages. As a result, the pain relief fluctuates between dosages corresponding to the peaks and valleys in the level of aspirin in the blood.

Many attempts have been made to develop timed-released pharmaceutical preparations which provide a more constant level of the drug in the blood over several hours.

One common approach is to microencapsulate aspirin, for example, with a capsule wall material which provides a slower dissolution rate than free aspirin. The early work in that regard is represented by U.S. Patent Nos. 3,155,590; 3,341,416; 3,488,418, and 3,531,418. Those patents, among others, disclose dispersing particles of aspirin in a hot cyclohexane solution containing ethyl cellulose and then introducing a phase-separation inducing agent, such as butyl rubber or polyethylene. Upon cooling, the aspirin particles become coated with ethyl cellulose. The coated particles are then admixed with tabletting excipients and formed into dosage-sized tablets. When ingested, the tablets disintegrate rapidly and the individual particles of encapsulated aspirin are dispersed in the stomach. The gastric juices slowly diffuse through the capsule walls, dissolve the aspirin, and the dissolved aspirin slowly diffuses or leaches out through the capsule walls into the body. Although the resultant blood level content is sustained to a measurable extent, the aspirin is diffused into the body rapidly enough so there is an initially high blood level content which decreases quite rapidly within a few hours. These dissolution properties yield undesirable blood aspirin concentration versus time curves.

In the first place, the time required to reach therapeutic levels after ingestion is longer for timed-release aspirin than for free aspirin. For this reason, it has been proposed that free aspirin be tableted with coated aspirin particles. See, for example, U.S. Patent No. 3,115,441 which discloses mixing aspirin particles having a series of coatings thereon with uncoated aspirin, and tableting so that the coated particles are entrapped in uncoated aspirin. Tablets made according to the method have the advantage of providing immediate relief because the free aspirin (which comprises the initial dosage) dissolves immediately upon ingestion. However, as with the other preparations discussed above, the tablet rapidly disintegrates in the stomach.

See also Guy U.S. Patent No. 4,025,613 where a multi-layered tablet is disclosed. One layer comprises aspirin coated with cellulose acetate phthalate and the other layer is free aspirin. However, as stated in Dunn, U.S. Patent No. 4,520,009, while aspirin tablets prepared by the process of Guy exhibit desirable in vitro release properties, processing difficulties are encountered in production runs. Dunn, then, is said to be an improvement in that large batch processing is allegedly made easier. In Dunn an admixture of aspirin, microcrystalline cellulose, cellulose acetate phthalate, plasticizer, corn starch and lubricant is compressed into tablet form. There is no microencapsulation as such in Dunn and, accordingly, the advantages of microencapsulated particles are foregone in favor of processing ease.

With microencapsulated particles, as discussed above, the dissolution rate decreases rapidly and the blood aspirin concentration at 2-3 hours must greatly exceed the therapeutic level in order to maintain adequate aspirin concentrations at 8 hours. As a result, efforts have been made to adjust the rate of dissolution and, thus, control the timing of sustained drug release. See, for example, Peters U.S. Patent No. 3,492,397 where the dissolution rate is said to be controlled by adjusting the wax/ethyl cellulose ratio of the applied spray coating. See also U.S. Patents No. 4,205,060 and 3,488,418 where it is indicated that the rate of dissolution can be controlled by varying the thickness of the coating.

Another method for providing an encapsulated pharmaceutical composition is discussed in published European patent Application No. 77,956, published May 4, 1983. EPO Publication No. 77,956 discloses the use of microcapsules containing a coated core material such as pharmaceutical compounds and foodstuffs. The coating is applied by dispersing the core material into a solution containing ethyl cellulose as the wall-forming material. A phase separation of the ethyl cellulose from the dispersion of core material is carried

2

out by cooling the dispersion. During this cooling, an enteric polymer material is incorporated into the ethyl cellulose coating walls by adding the enteric polymer material with stirring while the ethyl cellulose is still in the "gel" state. The enteric polymer material thus added penetrates, and is dispersed into the coating walls. When the microcapsules are administered, the release of the active compound does not generally occur in the stomach. However, the enteric polymer material is easily dissolved in the intestinal tract, thereby making the microcapsules porous. The porosity of the microcapsules promotes the rapid release of the active compound in the intestinal tract.

A similar approach is found in Japanese Patent Publication No. 12614/81, published March 23, 1981.

Japanese Publication No 12614/81 discloses an enteric protective coating composition which will not readily dissolve in acidic gastric juices, but rapidly (within minutes) dissolves at the pH found in the intestines. The enteric coating is an aqueous dispersion of, for example, hydroxy propyl methyl cellulose phthalate, a gelling agent such as diacetin, and hydroxy propyl methyl cellulose. See, also, Japanese Patent Publication No 11687/81, published March 16, 1981, which uses hydroxy propyl methyl cellulose phthalate as an enteric coating.

The systems described in the EPO and Japanese publications are essentially "delayed" release mechanisms. There is a delay of medicament release in the stomach, but once the coated medicament reaches the intestines, the release of medication is rapid. There is no sustained release of medication in the intestines.

FR-A-2287216 discloses a sustained release formulation for a medicament such as aspirin, comprising capsules containing the medicament in two distinct forms, namely a microencapsulated form and a micropulverised form of particle size 40 to 200 $\mu$m.

FR-A-2184902 and GB-A-1436714 disclose a medicinal preparation wherein the active ingredient is an iron or potassium compound, in the form of small pellets with a gastric-juice-resistant coating, generally based on cellulose compounds. An exemplified composition releases 15% of the active ingredient in one hour at gastric pH and 80% in one hour at pH 6.5.

The need thus remains for a sustained-release system which provides initial therapeutic levels of the drug; delays the delivery of another fraction of the drug to eliminate excess concentrations at 2-3 hours, and then, sustains the release of that delayed fraction to provide adequate drug levels for 8 or more hours.

According to a first aspect of the invention there is provided a sustained-release pharmaceutical preparation comprising an admixture of a) an uncoated or single walled water-soluble drug, and b) microcapsules of a dual walled coated medicament comprising a core of said drug in granular form, said granular core being coated with a microencapsular control coating at a weight ratio of drug to inner wall microencapsular control coating of from 2:1 to 20:1, said inner wall microencapsular control coating being one which will not dissolve or disperse readily in the intestines, but which permits release of said drug through said inner microencapsular coating and said inner wall coated drug, being coated with an outer wall enteric coating at a weight ratio of inner wall coated drug to outer wall enteric coating of from 4:1 to 12:1, said outer wall enteric coating being one which will not dissolve or dispense readily in the stomach, but which dissolves or disperses in the intestines whereby said dual walled coated medicament will slowly release said core drug in the intestines to provide adequate drug levels for 8 or more hours.

According to a further aspect of the invention there is provided a sustained-release pharmaceutical preparation comprising microcapsules of a dual walled coated medicament having a core of water soluble drug in granular form, an inner wall microencapsular control coating selected from ethyl cellulose, hydroxy propyl cellulose, carboxy methyl cellulose, and admixtures thereof, coated on said granular core drug at a weight ratio of drug to inner wall microencapsular control coating of from 2:1 to 20:1, and an outer wall enteric coating coated on said inner wall coated drug, selected from cellulose acetate phthalate, shellac, wax and phthalate or polyphthalate esters of film-forming polymers applied at a weight ratio of inner wall coated drug to outer wall enteric coating of from 4:1 to 12:1 whereby said dual walled coated medicament will slowly release said core drug in the intestines to provide adequate drug levels for 8 or more hours.

By enterically coating microcapsules, the release of core drug into the stomach is greatly impeded and the delivery of the drug is substantially delayed until the coated microcapsules reach the intestine. Delaying the delivery of part of the drug allows for incorporation of uncoated or single walled water-soluble drug into a pharmaceutical preparation to reduce the time required to reach therapeutic levels. The uncoated drug rapidly dissolves in the stomach and quickly enters the blood stream. The single walled drug begins to dissolve in the stomach and enter the blood stream in a controlled manner. In the intestines, the outer wall enteric coating film, membrane, or matrix dissolves or disperses in the intestinal fluid. However, the inner microcapsular control coating does not readily dissolve or disperse in the intestines. Rather, the drug is released in an enhanced controlled fashion through the inner microencapsular control coating, film, membrane, or matrix. Excess drug concentrations are minimized and steady long-term release of the drug is maximized.

The inner wall microencapsular control coating is preferably selected from the group consisting of ethyl cellulose, hydroxy propyl cellulose, and carboxy methyl cellulose. Most preferred is ethyl cellulose. Ethyl cellulose is a common microencapsular coating which will not readily dissolve or disperse in the stomach or intestines, but which permits release of the water-soluble drug through the capsule wall.

The outer wall enteric coating is preferably a microencapsular one such as cellulose acetate phthalate. Cellulose acetate phthalate is also a known coating material. A cellulose acetate phthalate outer wall enteric coating greatly impedes the release of the core drug at pH 1.1 as found in the stomach. But, cellulose acetate phthalate dissolves at pH 7.5 as found in the intestine to allow the release of the drug. Other enteric coatings may be used as long as they do not readily dissolve or disperse in the gastric juices of the stomach but do dissolve or disperse in the intestinal fluid of the intestines. For example, hydroxypropyl methyl cellulose phthalate, polyvinyl acetate phthalate, hydroxyethyl ethyl cellulose phthalate, cellulose acetate tetrahydrophthalate, acrylic resin, shellac, wax, or other film-forming materials which dissolve or disperse in the intestine but remain intact in the stomach are possible alternatives.

The core drug should be one which is reasonably water soluble so as to be slowly releasable in the intestines through the inner wall microencapsular control coating. Preferred are aspirin, acetaminophen, dextromethorphan hydrobromide, disopyramide phosphate and furosemide. Other usable water-soluble drugs include various vitamins, minerals, antibiotics, and other analgesics.

The water-soluble drug is preferably microencapsulated in granular form by a coacervation, spray coating or other process prior to having the outer wall enteric coating applied at a weight ratio of to core drug phase to inner wall phase of 2:1 to 20:1. The outer wall enteric coating is also preferably applied by a coacervation, spray coating or other process. The weight ratio of the microencapsulated core drug phase to the enteric coating phase is, 4:1 to 12:1 and most preferably from approximately 4:1 to 8:1. The resulting dual walled coated medicament has highly desirable release kinetics. In this form, the dual walled coated medicament will not release significant amounts of drug in the stomach. However, the outer wall enteric coating will dissolve or disperse in the intestines. Because the inner wall microencapsulated control coating remains, the drug is slowly and steadily released in the intestines.

The dual walled coated medicament may also be admixed with other fractions of free and/or timed-release drug. The admixture may be placed in either capsules or tablets along with other usual ingredients such as binders, fillers, lubricants, etc. In this form free drug is released immediately in the stomach. The dual walled coated medicament does not release drug in the stomach; but rather, in the intestines, drug is released slowly and steadily from the dual walled coated portion of the admixture by reason of the mechanism discussed above. The admixture, thus, provides for both immediate and sustained release of the drug.

The optimum sustained-release pharmaceutical preparation for 8 hour sustained-release aspirin, for example, has been determined to be 400-600 mg. of uncoated aspirin and 400-600 mg. of aspirin in 6:1 weight ratio cellulose acetate phthalate encapsulated 8:1 weight ratio ethyl cellulose microcapsules. A 975 mg. (active ingredient) admixture of this formulation produces a steady blood aspirin concentration versus time curve which rapidly reaches therapeutic levels, does not give excess blood concentration at any time, and maintains therapeutic levels for 8 hours.

Generally, the preferred analgesic therapeutic level of aspirin in the blood stream is between about 20 and 45 mg./l. Additional aspirin levels above 45 mg./l. are believed to have no additional analgesic effect. Further, there is an increased risk of toxicity associated with higher blood levels of aspirin.

Ordinary timed-release aspirin (tableted ethyl cellulose microencapsulated aspirin particles) containing 1300 mg. aspirin maintains therapeutic levels (i.e. above about 20 gm/l) for 8 hours, but gives an aspirin concentration greatly in excess of the therapeutic level 2-3 hours after ingestion of the tablets. Comparatively, the 25% reduction in dosage while maintaining therapeutic aspirin blood concentrations makes the sustained-release pharmaceutical preparation of the present invention therapeutically and/or economically attractive.

1300 mg. of an admixture containing 325-425 mg. of uncoated aspirin and 875-975 mg. aspirin in 6:1 weight ratio cellulose acetate phthalate encapsulated 8:1 weight ratio ethylcellulose microcapsules provides a 12 hour sustained release-aspirin formulation with the peak blood aspirin concentrations considerably lower than the peak blood aspirin concentration for Bayer timed-release aspirin, yet maintains therapeutic blood aspirin concentrations 12 hours after ingestion of the dosage.

By means of the present invention it is possible to provide a sustained-release pharmaceutical preparation which has desirable release kinetics and yet has a better therapeutic index and/or is more economical to produce. The dual walled coated medicament which may be used either alone or may be combined with other fractions of free and/or timed-release drug as a sustained-release pharmaceutical preparation.

The preferred dual walled coated medicament of the present invention is a granular aspirin core drug microencapsulated with an ethyl cellulose inner wall microencapsular control coating and a cellulose acetate phthalate outer wall enteric coating. The preferred 8 hour sustained-release pharmaceutical preparation is an admixture of that dual walled coated aspirin and uncoated aspirin particles, with an optimum formulation of 480 mg. uncoated aspirin and 495 mg. of aspirin in 6:1 weight ratio cellulose acetate phthalate encapsulated 12:1 weight ratio ethyl cellulose microcapsules.

The preferred sustained-release pharmaceutical preparation for 12 hour sustained release aspirin is an admixture of 325-425 mg. unencapsulated aspirin and 875-975 mg. of 6:1 weight ratio cellulose acetate phthalate encapsulated 8:1 weight ratio ethyl cellulose microcapsules.

Examples of other water-soluble drugs which may be used as the core drug include most preferably acetaminophen, furosemide, disopyramide phosphate, and dextromethorphan. In addition to these classes of water-soluble drugs others may also be used. For example vitamins, minerals, antibiotics, and other analgesics may be used as the core drug. As long as the drug has sufficient water solubility to be releasable in the intestines through the inner wall microencapsular control coating, is or can be made granular, and is capable of having the dual wall structure of the present invention applied to the granular drug. it is usable.

Other preferred inner wall microencapsular control coatings include hydroxy propyl cellulose and carboxy methyl cellulose. As mentioned, the inner wall microencapsular control coating should be one which does not readily dissolve or disperse in either the stomach or the intestines. It must, however, permit the aqueous intestinal fluids to diffuse through the capsule wall, dissolve the water-soluble core drug, and slowly diffuse or leach out through the capsule wall. It should also be a material which is preferably applicable by coacervation, spray coating or other processes to the granular drug. An ethyl cellulose capsule wall applied in a cyclohexane solution and coacervated by introduction of a phase-separation inducing agent (as taught by U.S. Patents No. 3,155,590; 3,341,416; 3,488,418 and 3,531,418, among others) is most preferred.

Cellulose acetate phthalate is the preferred outer wall enteric coating because it can be applied by coating processes (such as spray coating) or it can be applied by a coacervation process. Cellulose acetate phthalate is also particularly preferred because it will not readily dissolve or disperse at the low pH (around 1.1) of the gastric juices in the stomach. It remains relatively undissolved for over 2 hours under those conditions. And yet, at the higher pH (around 7.5) of the intestinal fluids found in the intestines cellulose acetate phthalate will dissolve or disperse. Other materials which may be used for such purposes are acrylic resin, shellac, wax, and phthalate or polyphthalate esters of film-forming polymers such as those already mentioned.

In a coacervation process cellulose acetate phthalate may be applied to the already encapsulated core drug by adding the ethyl cellulose encapsulated particles to cellulose acetate phthalate dissolved preferably in a buffer with a pH above 5.5, such as 1% aqueous solution of disodium hydrogen phosphate ($Na_2HPO_4$). A phase separation inducing agent, preferably a 10-40% aqueous solution of a soluble salt such as aqueous sodium sulfate, is used to coacervate the cellulose acetate phthalate and form the outer wall enteric coating. The preferred hardener for the cellulose acetate phthalate outer wall is acetic acid (HOAc); although, other inorganic or low molecular weight organic acids may be used. The type and amount of phase separation inducing agent and hardener is a function of the amount of cellulose acetate phthalate and the pH and strength of the buffer. Thus, the objective is to add a phase separation inducing agent to cause coacervation. Following coacervation the cellulose acetate phthalate encapsulated ethyl cellulose microcapsulate is hardened by reduction of the pH to below about 5.5, preferably to below about 4.0 pH. The dual wall microcapsules may then be washed and dried. The basic coacervation process is similar to the one reported by Merkle and Spieser in J. Pharm. Sci., 62:1444 (1973).

The weight ratio of the encapsulated core drug phase to the enteric coating phase is from 4:1 to 12:1. This ratio determines the thickness of the outer wall enteric coating. The weight ratio of the core drug phase to inner wall microencapsular coating phase is from 2:1 to 20:1. Table I below illustrates this as well as the degree to which the enteric coating prevents dissolution or dispersion in gastric juices.

Table I A

| pH 1.1 HCl/NaCl Buffer 4:1 CELLULOSE ACETATE PHTHALATE WEIGHT RATIO | | |
|---|---|---|
| ETHYL CELLULOSE WEIGHT RATIO | ELAPSED TIME | % DISSOLVED |
| 16:1 | 1 hr. | < 10% |
| 12:1 | 1 hr. | < 10% |
| 8:1 | 1 hr. | < 10% |
| 16:1 | 2 hrs. | < 10% |
| 12:1 | 2 hrs. | < 10% |
| 8:1 | 2 hrs. | < 10% |

Table I B

| pH 7.5 phosphate/NaOH buffer 4:1 CELLULOSE ACETATE PHTHALATE WEIGHT RATIO | | |
|---|---|---|
| ETHYL CELLULOSE WEIGHT RATIO | ELAPSED TIME | % DISSOLVED |
| 16:1 | 1 hr. | 60% |
| 12:1 | 1 hr. | 25% |
| 8:1 | 1 hr. | 10% |
| 16:1 | 2 hrs. | 75% |
| 12:1 | 2 hrs. | 50% |
| 8:1 | 2 hrs. | 15% |
| 16:1 | 3 hrs. | 90% |
| 12:1 | 3 hrs. | 70% |
| 8:1 | 3 hrs. | 20% |
| 16:1 | 4 hrs. | 95% |
| 12:1 | 4 hrs. | 80% |
| 8:1 | 4 hrs. | 25% |

The dissolution studies set forth in Table I were with a dual walled coated aspirin having an ethyl cellulose inner wall and a cellulose acetate phthalate outer wall. The tests were performed using the USP XX basket method. In each run a 750 mg. sample was placed in the basket rotated at 50 RPM in a 1 liter, 3-neck round bottom flask containing a buffer as set forth in Table I A at 37°C. After 2 hours the basket was removed from the flask, placed in a second flask containing a buffer as set forth in Table I B and rotated at 50 RPM for another 4 hours.

The buffer of Table I A is at the pH of gastric juices and the buffer of Table I B is at the pH of intestinal fluid. The dissolution procedure may also be performed using simulated digestive fluids. The composition of the buffers and digestive fluids is described in USP XX. In this instance simulated gastric juice is substituted for pH 1.1 buffer and simulated intestinal fluid is substituted for pH 7.5 buffer. Use of the digestive fluids is preferred if enzymes affect dissolution and is necessary if the coating contains lipid polymers or other enzymatically-degradable materials. However, neither of these conditions exists in the dissolution tests of Table I above (or Table II below) and performing the dissolutions in simulated digestive fluids does not significantly affect the dissolution rates of the dual walled coated medicament of the present invention.

The same basket dissolution method used in Table I was used for the tests of Table II below. Those runs were made with (A) Bayer timed-release aspirin, (B) 16:1 weight ratio ethyl cellulose microencapsulated aspirin capsules, and (C) dual walled coated aspirin having an inner wall of 12:1 weight ratio ethyl cellulose and an outer wall of 4:1 weight ratio cellulose acetate phthalate.

## Table II A
## pH 1.1 HCl/NaCl buffer

| RUN | ELAPSED TIME | % DISSOLVED |
|-----|--------------|-------------|
| A | 1 hr. | 40% |
| B | 1 hr. | 20% |
| C | 1 hr. | < 10% |
| A | 2 hrs. | 60% |
| B | 2 hrs. | 35% |
| C | 2 hrs. | < 10% |

## Table II B
## pH 7.5 phosphate/NaOH buffer

| RUN | ELAPSED TIME | % DISSOLVED |
|-----|--------------|-------------|
| A | 1 hr. | 90% |
| B | 1 hr. | 65% |
| C | 1 hr. | 30% |
| A | 2 hrs. | 100% |
| B | 2 hrs. | 80% |
| C | 2 hrs. | 50% |
| A | 3 hrs. | 100% |
| B | 3 hrs. | 85% |
| C | 3 hrs. | 65% |
| A | 4 hrs. | 100% |
| B | 4 hrs. | 90% |
| C | 4 hrs. | 85% |

Table II shows that the dual walled coated medicament of the present invention (run C) effectively delays dissolution (and drug release) until the basket is placed in pH 7.5 buffer and even then, the dissolution of run C is slow and steady. On the other hand, runs A and B rapidly released aspirin at pH 1.1 and were nearly completely dissolved shortly after introduction into the pH 7.5 buffer. Again, performing the dissolution studies in simulated digestive fluids does not significantly effect the dissolution rates of any of runs A, B or C. The preferred dual walled coated medicament and sustained-release pharmaceutical preparations may be prepared as best illustrated in the following examples (the trade marks as used therein being acknowledged as such).

EXAMPLE 1

A twelve hour sustained-release pharmaceutical preparation utilizing the dual wall coating of the present invention was prepared as follows.

14.25 gr. ethyl cellulose, 48-49.5% ethoxylated, viscosity 100 (Dow Ethocel Premium) and 9.5 gr. polyethylene (Kodak Epolene C-10) were added to 600 ml of cyclohexane. The mixture was refluxed for 20 minutes with stirring to form a solution. 114 gr. of aspirin, USP No. 40 crystals (Dow), were added to the solution and the mixture was cooled to 20°C in 20 minutes. The mixture was filtered and the filter cake was washed with 500 ml cyclohexane. The resulting aspirin ethyl cellulose microcapsules were dried by sieving through a 20 mesh screen 3 times.

72 gr. of the aspirin ethyl cellulose microcapsules were added to a stirred solution of 12 gr. cellulose acetate phthalate (Eastman C.A.P.) and 4.5 gr. $Na_2HPO_4$ in 585 gr. water at 40°C. While stirring, 420 gr. of 30% aqueous sodium sulfate were added dropwise over a 4.5 minute period. The outer wall enteric coating was fixed by the immediate addition of 90 ml of 20% acetic acid. The resulting dual walled microcapsules were filtered, washed with 750 ml of 2% acetic acid and dried for 20 minutes in a fluidized bed drier at 25°C. The aspirin content was found to be 77.4%. The above described procedure produced 6:1 weight ratio cellulose acetate phthalate encapsulated 8:1 weight ratio ethyl cellulose aspirin capsules. The weight ratio is determined by the amounts of polymer and drug used in preparation of the microcapsules. Thus 114 gr. of aspirin encapsulated with 14.25 gr. of ethyl cellulose inner wall material equals a weight ratio of 114 to 14.25 or 8 to 1. Likewise 72 gr. of aspirin ethyl cellulose microcapsules encapsulated with 12 gr. of cellulose acetate phthalate equals a weight ratio of 72 to 12 or 6 to 1.

38.00 grams of the dual walled microcapsule aspirin were mixed with 11.92 gr. USP aspirin powder (J.J. Baker). Size 00 gelatin capsules were filled with the mixture to contain 433 mg. aspirin, 125 mg. of which was free aspirin and 308 mg. of which was dual wall encapsulated aspirin. Clinical trials were performed with two capsules of the aspirin compound of the instant invention. The two capsules together contained 867 mg. of aspirin (250 mg. free aspirin and 617 mg. of dual wall encapsulated aspirin). The trials were conducted against 1300 mg. Bayer Timed Release aspirin in a six subject cross-over study. As can be seen from the data in Tables IIIA and IIIB, the formulation containing dual walled microcapsules and free aspirin provided nearly constant blood salicylate concentrations.

## TABLE III A

### 867 mg. uncoated aspirin/dual wall coated aspirin mix

| ELAPSED TIME | BLOOD ASA CONCENTRATION (mg./l.) |
|---|---|
| 1 hr. | 18 |
| 2 hrs. | 19 |
| 3 hrs. | 21 |
| 4 hrs. | 21 |
| 5 hrs. | 19 |
| 6 hrs. | 21 |
| 7 hrs. | 19 |
| 8 hrs. | 17 |
| 9 hrs. | 16 |
| 10 hrs. | 15 |
| 11 hrs. | 15 |
| 12 hrs. | 15 |
| 13 hrs. | 14 |
| 14 hrs. | 13 |
| 15 hrs. | 12 |
| 16 hrs. | 11 |

## TABLE III B

### 1300 mg. Bayer, ethyl cellulose coated time release aspirin

| ELAPSED TIME | BLOOD ASA CONCENTRATION (mg./l.) |
|---|---|
| 1 hr. | 34 |
| 2 hrs. | 57 |
| 3 hrs. | 68 |
| 4 hrs. | 80 |
| 5 hrs. | 86 |
| 6 hrs. | 77 |
| 7 hrs. | 67 |
| 8 hrs. | 57 |
| 9 hrs. | 54 |
| 10 hrs. | 51 |
| 11 hrs. | 46 |
| 12 hrs. | 41 |
| 13 hrs. | 37 |
| 14 hrs. | 32 |
| 15 hrs. | 28 |
| 16 hrs. | 24 |

The Bayer Timed-Release aspirin provided wide variations in blood salicylate concentrations, gave a high peak concentration and saturated the body's salicylate elimination mechanism to maintain high salicylate concentrations.

EXAMPLE 2

25.00 gr. of the dual-walled microcapsules from Example 1 were mixed with 18.76 gr. of powdered aspirin. Gelatin capsules were filled with 375 mg. of the mixture to contain 325 mg. aspirin, 160 g. of which was free aspirin, and 165 g. of which was dual wall microencapsulated aspirin of the present invention. Clinical trials were performed with 975 mg. of aspirin (three, 375 mg. capsules, together containing 480 mg. free aspirin and 495 mg. of dual wall microencapsulated aspirin) in the capsules and 975 mg. Bayer Regular Aspirin. As can be seen in TABLES IV-A and IV-B the sustained-release formulation gave more constant blood salicylate concentrations than plain aspirin with peak blood levels half of that produced by free aspirin.

## TABLE IV A

## 975 mg. uncoated aspirin/dual walled coated aspirin mix

| ELAPSED TIME | BLOOD ASA CONCENTRATION (mg./l.) |
|---|---|
| 1 hr. | 30 |
| 2 hrs. | 34 |
| 3 hrs. | 34 |
| 4 hrs. | 31 |
| 5 hrs. | 29 |
| 6 hrs. | 25 |
| 8 hrs. | 18 |
| 10 hrs. | 14 |
| 12 hrs. | 13 |

## TABLE IV B

## 975 mg. Regular Bayer Aspirin

| ELAPSED TIME | BLOOD ASA CONCENTRATION (mg./l.) |
|---|---|
| 1 hr. | 63 |
| 2 hrs. | 68 |
| 3 hrs. | 62 |
| 4 hrs. | 56 |
| 5 hrs. | 51 |
| 6 hrs. | 42 |
| 8 hrs. | 26 |
| 10 hrs. | 16 |
| 12 hrs. | 7 |

EXAMPLE 3

A solution was formed with 200 ml water, 30 ml. polyethylene glycol 400, 2 gr. $Na_2HPO_4$ and 4 gr. polyvinyl acetate phthalate (Canada Packers). The pH was adjusted to 4.85 with 0.1 N HCl. 16 gr. of 8:1 phase ratio aspirin ethyl cellulose microcapsules prepared in the procedure in Example I were added to the solution at 55°C. 200 gr. of 30% aqueous sodium sulfate were added over a 4 minute period.

A silica dispersion was then prepared in the following manner. A mixture of 2 gr. aerosil R972 hydrophobic silical powder (Degussa, Teterboro, NJ) and 10 drops, 2% Naccanol in water (Stepan, Northfield, Illinois) was dispersed in 300 ml water and the pH was lowered to 3.0 with 0.1 N HCl. This silica dispersion (at 25°C) was added to the mixture containing the microcapsules. The pH was further reduced to 2.75 and the mixture was cooled at 25°C. The microcapsules were filtered, washed with 500 ml water acidified to pH 2.75 with 0.1 N HCl, and dried in a fluidized bed drier.

The same basket dissolution method used in conjunction with the data set forth in Tables I-A and I-B was used to test the polyvinyl acetate phthalate coated capsules produced in this example. The data from the tests of the polyvinyl acetate phthalate coated capsules is set forth in Tables V-A and V-B. Table V-A shows that the polyvinyl acetate phthalate outer wall effectively precludes dissolution (and drug release) in the pH 1.1 buffer. As discussed above, the pH 1.1 buffer simulates conditions in the stomach.

## TABLE V-A

## PH 1.1 HCl/NaCl BUFFER

### POLYVINYL ACETATE PHTHALATE OUTER MICROENCAPSULAR WALL

| ELAPSED TIME | % DISSOLVED |
|---|---|
| 1 hr. | 1 |
| 2 hrs. | 2 |

## TABLE V B
### pH 7.5 PHOSPHATE BUFFER
### POLYVINYL ACETATE PHTHALATE OUTER MICROENCAPSULAR WALL

| ELAPSED TIME | % DISSOLVED |
|---|---|
| 1 hr. | 26 |
| 2 hrs. | 44 |
| 4 hrs. | 68 |

EXAMPLE 4

2:1 weight ratio furosemide-ethyl cellulose microcapsules were prepared by the phase separation of ethyl cellulose from cyclohexane containing polyethylene. A second microencapsular wall of cellulose acetate phthalate was applied by the coacervation method described in Example 1.

The same basket dissolution method used to produce the data set forth in Tables I-A and I-B was used for the tests reported in Tables VI-A and VI-B below. Table VI-A shows that the dual-walled microcapsules do not release any substantial amount of furosemide in pH 1.1 buffer. Table VI-B shows that a slow dissolution of the drug is achieved in pH 7.5 buffer.

### Table VI A

| pH 1.1 HCl/NaCl BUFFER FUROSEMIDE DUAL-WALLED MICROENCAPSULAR DRUG | |
|---|---|
| ELAPSED TIME | % DISSOLVED |
| 1 hr. | <1 |
| 2 hrs. | <1 |

## TABLE VI B

### pH 7.5 BUFFER

### FUROSEMIDE DUAL-WALLED MICROENCAPSULAR DRUG

| ELAPSED TIME | % DISSOLVED |
|---|---|
| 1 hr. | 47 |
| 2 hrs. | 62 |
| 4 hrs. | 76 |
| 6 hrs. | 82 |

Tables VII-A shows computer predicted blood furosemide concentration versus time curve for 80 mg. free furosemide. Table VII-B shows a computer predicted blood concentration versus time curve for a sustained release formulation composed of 20 mg. free furosemide and 60 mg. of furosemide in the dual walled microcapsules of Example IV. Free furosemide reaches a high peak concentration after 1 hour then rapidly declines. The sustained-release formulation maintains a generally consistent blood furosemide concentration.

## TABLE VII A

### 80 mg. Free Furosemide

### PREDICTED BLOOD FUROSEMIDE CONCENTRATION

| ELAPSED TIME | FUROSEMIDE CONCENTRATION |
|---|---|
| 1 hr. | 2.2 mg./l. |
| 2 hrs. | 1.3 mg./l. |
| 3 hrs. | 0.6 mg./l. |
| 4 hrs. | 0.3 mg./l. |
| 6 hrs. | 0.1 mg./l. |
| 8 hrs. | 0.1 mg./l. |

TABLE VII B

| 80 mg. Sustained Release PREDICTED BLOOD FUROSEMIDE CONCENTRATION | |
|---|---|
| ELAPSED TIME | FUROSEMIDE CONCENTRATION |
| 1 hr. | 0.6 mg./l. |
| 2 hrs. | 0.4 mg./l. |
| 3 hrs. | 0.7 mg./l. |
| 4 hrs. | 0.8 mg./l. |
| 6 hrs. | 0.4 mg./l. |
| 8 hrs. | 0.2 mg./l. |

EXAMPLE 5

Capsules of 40 mesh (420 $\mu$m) ethyl cellulose encapsulated aspirin were prepared by the coacervation process described in U.S. Patent No. 3,155,590. 8.0 grams of capsules were added to 100 grams of a 2% solution of cellulose acetate phthalate in a 1% aqueous solution of $KNaHPO_4$ and stirred at 55°C. While stirring 20 ml. of 20% aqueous sodium sulfate solution was added to the system in 30 seconds. This was immediately followed by the dropwise addition of 40 ml. of 20% aqueous sodium sulfate in 4.5 minutes. Coacervation resulted in coating of the ethyl cellulose encapsulated aspirin capsules with an outer wall of cellulose acetate phthalate at a weight ratio of 4:1. The outer walled enteric coating was fixed by the addition of 5 ml of 14% HOAc in 1 minute. The resulting dual walled coated capsules were washed with 2% HOAc and dried for 1 hour on a 60 mesh (250 $\mu$m) screen in a fluidized bed drier.

Studies were undertaken to determine the optimum formulation for an 8-hour sustained-release pharmaceutical preparation utilizing the dual walled coated aspirin of this example. It was determined that preferred is 480 mg. uncoated aspirin and 495 mg. of aspirin in the 4:1 weight ratio cellulose acetate phthalate encapsulated 8:1 weight ratio ethyl cellulose aspirin capsules. The optimum formulation was determined by choosing the combination which had the blood curve with the best least-square curve fit to a constant 30 mg/liter blood concentration.

The predicted blood curve for the optimum formulation is set forth in Table VIII below.

TABLE VIII

| 975 mg. uncoated aspirin/dual walled coated aspirin mix | |
| --- | --- |
| ELAPSED TIME | BLOOD ASA CONCENTRATION (mg./l.) |
| 1 hr. | 25 |
| 2 hrs. | 29 |
| 3 hrs. | 30 |
| 4 hrs. | 34 |
| 5 hrs. | 33 |
| 6 hrs. | 33 |
| 7 hrs. | 25 |
| 8 hrs. | 20 |

EXAMPLE 6

This example illustrates the formation of the dual-walled microcapsules of this invention by a spray coating technique.

800 gm of a granulated aspirin (Asagran 1640, Monsanto) was placed in the Wurster bowl of a Uniglatt fluid bed spray coating machine. The inner microencapsular wall was applied by spraying onto the aspirin a dispersion of 200 gm Aquacoat aqueous ethylcellulose dispersion (FMC Corporation), 14.4 gm Myvacet 9-40 acetylated monoglycerides (Eastman Chemical Products) and water to dilute the solids content to 20% of the weight of the dispersion. 221 gm of the dispersion was sprayed onto the aspirin to yield microcapsules with a weight ratio of 19:1.

The outer microencapsular wall was also applied by spray coating. The second coating dispersion was prepared by adding to 218 gm of water, slowly stirred, in a blender, 12 gm of Talc (2755 Lo-micron Talc USP, Whittaker), 0.75 gm antifoam agent (Medical Antifoam AF Emulsion, Dow Corning) and a solution of 10 gm polyethylene glycol 8000 (Baker) and 2 gm polyethylene glycol 1000 (Baker) in 108 gm water. The blender was then run at high speed for 1 minute. This suspension was poured into 120 gm of Eudragit L 30 D aqueous acrylic resin dispersion (Rohm Pharma). 209 gm of this dispersion was sprayed onto 600 gm of of ethylcellulose encapsulated aspirin to yield dual walled microcapsules with an ethylene aspirin microcapsule to enteric coating weight ratio of 19:1.

The same basket dissolution method used in conjunction with the data set forth in Tables I-A and I-B was used to test the dual walled microcapsules prepared in this example. The data from the tests of dual walled microcapsules prepared in this example. The data from the tests of dual walled microcapsules prepared by spray coating is set forth in Tables IX-A and IX-B. Table IX-A shows that the outer wall effectively precludes dissolution (and drug release) in the pH 1.1 buffer. As discussed above, the pH 1.1 buffer simulates conditions in the stomach. Table IX-B shows that the aspirin is released in a controlled manner in the pH 7.5 buffer which simulates conditions in the intestine.

TABLE IX-A

pH 1.1 HCl/NaCl Buffer

Dual Walled Microcapsules Prepared by Spray Coating

| ELAPSED TIME | % DISSOLVED |
| --- | --- |
| 1 hr. | <5 |
| 2 hrs. | <5 |

TABLE IX-B

pH 7.5 Phosphate Buffer

Dual Walled Microcapsules Prepared by Spray Coating

| ELAPSED TIME | % DISSOLVED |
| --- | --- |
| 1 hr. | 23 |
| 2 hrs. | 35 |
| 4 hrs. | 52 |

**Claims**

1. A sustained-release pharmaceutical preparation comprising an admixture of
   a) an uncoated or single walled water-soluble drug, and
   b) microcapsules of a dual walled coated medicament comprising a core of said drug in granular form, said granular core being coated with a microencapsular control coating at a weight ratio of drug to inner wall microencapsular control coating of from 2:1 to 20:1, said inner wall microencapsular control coating being one which will not dissolve or disperse readily in the intestines, but which permits release of said drug through said inner microencapsular coating and said inner wall coated drug being coated with an outer wall enteric coating at a weight ratio of inner wall coated drug to outer wall enteric coating of from 4:1 to 12:1, said outer wall enteric coating being one which will not dissolve or dispense readily in the stomach, but which dissolves or disperses in the intestines whereby said dual walled coated medicament will slowly release said core drug in the intestines to provide adequate drug levels for 8 or more hours.

2. A sustained-release pharmaceutical preparation according to claim 1 wherein said inner wall microencapsular control coating comprises ethyl cellulose, hydroxypropyl cellulose or carboxymethyl cellulose.

3. A sustained-release pharmaceutical preparation according to claim 1 or claim 2 wherein said outer wall enteric coating comprises cellulose acetate phthalate, hydroxypropylmethyl cellulose phthalate, polyvinyl acetate phthalate, hydroxyethyl ethyl cellulose phthalate, cellulose acetate tetrahydrophthalate, acrylic resin, shellac or wax.

4. A sustained-release pharmaceutical preparation according to any preceding claim wherein said drug is selected from aspirin, acetaminophen, dextromethorphan, hydrobromide, disopyramide phosphate and furosemide.

5. A sustained-release pharmaceutical preparation according to claim 3 wherein said drug is granular aspirin, said inner wall microencapsular control coating is ethyl cellulose, and said outer wall enteric coating is cellulose acetate phthalate.

6. A sustained-release pharmaceutical preparation according to claim 5 wherein said uncoated aspirin is present in the amount of 400-600 mg and said dual walled coated aspirin is present in the amount of 400-600 mg.

7. A sustained-release pharmaceutical preparation according to claim 5 wherein said uncoated aspirin is present in the amount of 325-425 mg and said dual walled coated aspirin is present in the amount of 875-975 mg.

8. A sustained-release pharmaceutical preparation comprising microcapsules of a dual walled coated medicament having:
a core of water soluble drug in granular form,
an inner wall microencapsular control coating selected from ethyl cellulose, hydroxy propyl cellulose, carboxy methyl cellulose, and admixtures thereof, coated on said granular core drug at a weight ratio of drug to inner wall microencapsular control coating of from 2:1 to 20:1, and
an outer wall enteric coating coated on said inner wall coated drug, selected from cellulose acetate phthalate, shellac, wax and phthalate or polyphthalate esters of film-forming polymers applied at a weight ratio of inner wall coated drug to outer wall enteric coating of from 4:1 to 12:1 whereby said dual walled coated medicament will slowly release said core drug in the intestines to provide adequate drug levels for 8 or more hours.

9. A dual walled coated medicament according to claim 8 wherein said core drug is selected from aspirin, acetominophen, dextromethorphan hydrobromide, disopyramide phosphate and furosemide.

10. A dual walled coated medicament according to claim 9 wherein said core drug is granular aspirin.

11. A dual walled coated medicament according to any one of claims 8 to 10 wherein said outer wall enteric coating comprises microencapsular cellulose acetate phthalate.

12. A dual walled coated medicament according to any one of claims 8 to 11 wherein said inner wall microencapsular control coating comprises ethyl cellulose.

13. A method for preparing a sustained-release pharmaceutical preparation comprising:
a) adding ethyl cellulose encapsulated granular aspirin coated at a weight ratio of aspirin to ethyl cellulose of 2:1 to 20:1, to a solution containing dissolved cellulose acetate phthalate, at a weight ratio of ethyl cellulose encapsulated aspirin to cellulose acetate phthalate of from 4:1 to 12:1;
(b) adding a phase separation inducing agent whereby coacervation occurs and produces a dual walled coated aspirin having an ethyl cellulose inner wall and a cellulose acetate phthalate outer wall,
(c) hardening said cellulose acetate phthalate outer wall,
(d) washing and drying said dual walled coated aspirin and
(e) admixing said dual walled coated aspirin with uncoated aspirin.

14. The method of claim 13 wherein said cellulose acetate phthalate containing solution is buffered at a pH greater than 5.5.

15. The method of claim 14 wherein said phase separation inducing agent is 10-40% aqueous sodium sulfate.

16. The method of claim 15 wherein said hardener is acetic acid.

17. The method of claim 16 wherein said pH is reduced to below 4 on addition of said hardener.

**Patentansprüche**

1. Arzneimittel mit verzögerter Freigabe, **gekennzeichnet durch** eine kombinierende Mischung von -
a) einem unbeschichteten oder einwandigen wasserlöslichen Medikament und
b) Mikrokapseln eines doppelwandig umhüllten Arzneimittels, bestehend aus einem Kern dieses Medikamemts in feinkörniger Form, wobei dieser feinkörnige Kern mit einer mikrokapselförmigen, die Freigabe steuernden inneren Umhüllung in einem Gewichtsverhältnis des Medikaments zu der Umhüllung von 2:1 bis 20:1 beschichtet ist, und diese die innere Wandung bildende freigabesteuern-

EP 0 239 361 B1

de Umhüllung eine ist, die sich nicht sogleich in den Därmen auflöst oder dispergiert, sondern die Freigabe des Medikaments durch die mikrokapselförmige Umhüllung erlaubt, und wobei die die Umhüllunmg des Medikaments bildende Innenwandung als Außenwandung eine enterische Umhüllung in einem Gewichtsverhältnis der inneren Hülle zur äußeren Hülle von 4:1 bis 12:1 aufweist, und die die Außenwandung bildende enterische Umhüllung eine ist, die sich nicht sogleich im Magen auflöst oder dispergiert, sonderm sich erst in den Därmen auflöst oder dispergiert, wodurch das doppelwandig umhüllte Arzneimittel das Kern-Medikament in einer adequaten Dosis über einen Zeitraum von 8 and mehr Stunden langsam in den Därmen freigibt.

2.  Arzneimittel mit verzögerter Freigabe nach Anspruch 1, **dadurch gekennzeichnet,** daß die freigabesteuernde innere Umhüllung aus Äthylzelluslose, Hydroxypropylzellulose oder Carboxymethylzellulose besteht.

3.  Arzneimittel mit verzögerter Freigabe, **dadurch gekennzeichnet,** daß die äußere enterische Umhüllung aus Zelluloseazetatphthalat, Hydroxypropylmethyl-Zellulosephthalat, Polyvinylazetatphthalat, Hydroäthyl-Äthylzellulosephthalat, Zelluloseazetat-Tetrahydrophthalat, Acrylharz, Schallack oder Wachs besteht.

4.  Arzneimittel mit verzögerter Freigabe nach einem der vorhergehenden Ansprüche, **dadurch gekennzeichnet,** daß das Medikament ausgewählt ist aus Aspirin, Acetaminophen, Dextrometorphan-Hydrobromid, Disopyramidphosphat und Furosemid.

5.  Arzneimittel mit verzögerter Freigabe nach Anspruch 3, **dadurch gekennzeichnet,** daß das Medikament ein feinkörniges Aspirin, die freigabesteuernde mikrokapselförmige innere Umhüllung Äthylzellulose, und die äußere enterische Umhüllung Zelluloseazetatphthalat ist.

6.  Arzneimittel mit verzögerter Freigabe nach Anspruch 5, **dadurch gekennzeichnet,** daß das unbeschichtete Aspirin in einer Menge von 400-600 mg und das doppelwandig umhüllte Aspirin in einer Menge von 400-600 mg vorhanden ist.

7.  Arzneimittel mit verzögerter Freigabe nach Anspruch 5, **dadurch gekennzeichnet,** daß das unbeschichtete Aspirin in einer Mange von 325-425 mg und das doppelwandig umhüllte Aspirin in einer Mange von 875-975 mg vorhanden ist.

8.  Arzneimittel mit verzögerter Freigabe, **gekennzeichnet durch** Mikrokapseln eines doppelwandig umhüllten Medikaments, mit -
    einem Kern aus einem wasserlöslichen Medikament in feinkörniger Form,
    einer mikrokapselförmigen die Freigabe steuernden inneren Umhüllung, ausgewählt aus Äthylzellulose, und Beimischungen derselben, aufgetragen auf dieses feinkörnige Kernmedikament in einem Gewichtsverhältnis des Medikaments zur mikrokapselförmigen freigabesteuernden inneren Umhüllung von 2:2 bis 20:1, und
    einer äußeren enterischen Umhüllung, aufgetragen auf die innere Umhüllung des Medikaments, ausgewählt aus Zelluloseazetatphthalat, Schellack, Wachs und Phthalat-oder Polyphthalatestern von filmbildenden Polymerisaten in einem Gewichtsverhältnis des inneren umhüllten Medikaments zur äußeren enterischen Umhüllung von 4:1 bis 12:1, wodurch das doppelwandig umhüllte Arzneimittel das Kernmedikament in einer adequaten Dosis über einen Zeitraum von 8 und mehr Stunden langsam in den Därmen freigibt.

9.  Doppelwandig umhülltes Arzneimittel nach Anspruch 8, **dadurch gekennzeichnet,** daß das Kernmedikament ausgewählt ist aus Aspirin, Acetaminophen, Dextrometorphan-Hydrobromid, Disopyramidphosphat und Furosemid.

10. Doppelwandig umhülltes Arzneimittel nach Anspruch 9, **dadurch gekennzeichnet,** daß das Kernmedikament ein feinkörniges Aspirin ist.

11. Doppelwandig umhülltes Arzneimittel nach einem der Ansprüche 8 bis 10, **dadurch gekennzeichnet,** daß die äussere enterische Umhüllung aus mikrokapselförmigen Zelluloseazetatphthalat besteht.

16

**12.** Doppelwandig umhülltes Arzneimittel nach einem der Ansprüche 8 bis 11, **dadurch gekennzeichnet,** daß die freigabesteuernde mikrokapselförmige innere Umhüllung aus Äthylzellulose besteht.

**13.** Verfahren zur Aufbereitung eines Arzneimittels mit verzögerter Freigabe, **gekennzeichnet durch -**

a) Zuführung des mit Äthylzellulose vergekapselten feinkörnigen Aspirins, in einem Gewichtsverhältnis des Aspirins zur Äthylzellulose von 2:1 bis 20:1, zu einer aufgelöstes Zelluloseazetatphthalat enthaltenden Lösung, in einem Gewichtsverhältnis des mit Äthylzellulose verkapselten Aspirins zum Zelluloseazetatphthalat von 4:1 bis 12:1;

b) Zusätzen eines phasentrennenden Primäragens, wodurch Koazervation eintritt und ein doppelwandig umhülltes Aspirin mit einer inneren Umhüllung aus Äthylzellulose und einer äußeren Umhüllung aus Zelluloseazetatphthalat erzeugt wird;

c) Härtung der aus Zelluloseazetatphthalat bestehenden äußeren Umhüllung;

d) Waschen und Trocknen des doppelwandig umhüllten Aspirins, und

e) Mischen des doppelwandig umhüllten Aspirins mit unbeschichteten Aspirin.

**14.** Verfahren nach Anspruch 13, **dadurch gekennzeichnet,** daß die Zelluloseazetatphthalat enthaltende Lösung bei einem pH-Wert größer als 5.5 gepuffert ist.

**15.** Verfahren nach Anspruch 14, **dadurch gekennzeichnet,** daß das phasentrennende Primäragens 10-40%iges wässeriges Natriumsulfat ist.

**16.** Verfahren nach Anspruch 15, **dadurch gekennzeichnet,** daß der Härter Essigsäure ist.

**17.** Verfahren nach Anspruch 16, **dadurch gekennzeichnet,** daß der pH-Wert beim Zusätzen des Härters unter 4 reduziert wird.

**Revendications**

**1.** Une préparation pharmaceutique à effet retenu comprenant en mélange :

a) une substance active non enrobée ou à simple paroi, et

b) des microcapsules d'un médicament enrobé d'une double paroi, comprenant un noyau de ladite substance active sous forme granulaire, ledit noyau granulaire étant enrobé avec un revêtement de microencapsulation de contrôle selon un rapport pondéral entre la substance active et le revêtement de microencapsulation de contrôle constituant la paroi interne compris entre 2/1 et 20/1, ledit revêtement de microencapsulation de de paroi interne de contrôle étant d'un type qui ne se dissout ni ne se disperse facilement dans les intestins, mais qui permet la libération de ladite substance active à travers ledit revêtement de microencapsulation interne, et ladite substance active enrobée d'une paroi interne étant enrobée avec un revêtement entérique constituant la paroi externe selon un rapport pondéral entre ladite substance active enrobée d'une paroi interne et le revêtement entérique de paroi externe compris entre 4/1 et 12/1, ledit revêtement entérique de paroi externe étant d'un type qui ne se dissout ni ne se disperse facilement dans l'estomac, mais se dissout ou se disperse dans les intestins, afin que ledit médicament enrobé d'une double paroi libère doucement ladite substance active du noyau dans les intestins pour procurer des niveaux de substance active corrects pendant huit heures ou plus.

**2.** Une préparation pharmaceutique à effet retenu selon la revendication 1, dans laquelle ledit revêtement de microencapsulation de paroi interne de contrôle comprend de l'éthylicellulose, de la cellulose hydroxypropylique ou de la cellulose carboxyméthylique.

**3.** Une préparation pharmaceutique à effet entretenu selon la revendication 1 ou 2, dans laquelle ledit revêtement entérique de paroi externe comprend du phtalate d'acétocellulose, du phtalate de cellulose hydroxypropylméthylique, du phtalate de polyvinylacétate, du phtalate de cellulose éthyl-hydroxyéthylique, du tétrahydrophtalate d'acétocellulose, de la résine acrylique, du shellac ou de la paraffine.

**4.** Une préparation pharmaceutique à effet retenu selon l'une des revendications précédentes, dans laquelle ladite substance active est choisie parmi l'aspirine, l'acétaminophène, le dextrométhorphan, l'hydrobromure, le phosphate de dispyramide et le furosémide.

5. Une préparation pharmaceutique à effet retenu selon la revendication 3, dans laquelle ladite substance active est de l'aspirine granulaire, ledit revêtement de de microencapsulation de paroi interne de contrôle est de l'éthylcellulose et ledit revêtement entérique de paroi externe est du phtalate d'acétocellulose.

6. Une préparation pharmaceutique à effet retenu selon la revendication 5, dans laquelle ladite aspirine non enrobée est présente en quantité de 400 à 600 mg et ladite aspirine enrobée d'une double paroi est présente en quantité de 400 à 600 mg.

7. Une préparation pharmaceutique à effet retenu selon la revendication 5, dans laquelle l'aspirine non enrobée est présente en quantité de 325 à 425 mg et ladite aspirine enrobée d'une double paroi est présente en quantité de 875 à 975 mg.

8. Une préparation pharmaceutique à effet retenu comprenant des microcapsules d'un médicament enrobé d'une double paroi comportant :
   un noyau de substance active soluble dans l'eau sous forme granulaire,
   un revêtement de microencapsulation constituant la paroi interne choisi parmi l'éthylcellulose, la cellulose hydroxypropylique, la cellulose carboxyméthylique, et des mélanges de celles-ci, enduit sur ladite substance active sous forme de noyau granulaire, selon un rapport pondéral entre la substance active et le revêtement de microencapsulation de paroi interne de contrôle compris entre 2/1 et 20/1, et
   un revêtement entérique constituant la paroi externe enduit sur ladite substance active enrobée d'une paroi interne, choisi parmi le phtalate d'acétocellulose, le shellac, la paraffine et des esters phtaliques ou polyphtaliques de polymères filmogènes, appliqué selon un rapport pondéral entre la substance active enrobée d'une paroi interne et le revêtement entérique de paroi externe compris entre 4/1 et 12/1, afin que le médicament enrobé d'une double paroi libère doucement ladite substance active du noyau dans les intestins pour procurer des niveaux de substance active corrects pendant huit heures ou plus.

9. Un médicament enrobé d'une double paroi selon la revendication 8, dans lequel ladite substance active du noyau est choisie parmi l'aspirine, l'acétominophène, le dextrométhorphan, l'hydrobromure, le phosphate de disopyramide et le furosémide.

10. Un médicament enrobé d'une double paroi selon la revendication 9, dans lequel ladite substance active du noyau est de l'aspirine granulaire.

11. Un médicament enrobé d'une double paroi selon l'une quelconque des revendications 8 à 10, dans lequel ledit revêtement entérique de paroi externe comprend du phtalate d'acétocellulose de microencapsulation.

12. Un médicament enrobé d'une double paroi selon l'une quelconque des revendications 8 à 11, dans lequel ledit revêtement de microencapsulation de paroi interne de contrôle comprend de l'éthylcellulose.

13. Un procédé pour préparer une préparation pharmaceutique à effet retenu comprenant les étapes suivantes :
   a) ajouter de l'aspirine granulaire encapsulée d'éthylcellulose selon un rapport pondéral entre l'aspirine et l'éthylcellulose compris entre 2/1 et 20/1, à une solution contenant du phtalate d'acétocellulose dissous, selon un rapport pondéral entre l'aspirine encapsulée d'éthylcellulose et le phtalate d'acétocellulose compris entre 4/1 et 12/1 ;
   b) ajouter un agent inducteur de séparation de phase afin de provoquer la coacervation et produire une aspirine enrobée d'une double paroi avec une paroi interne en éthylcellulose et une paroi externe en phtalate d'acétocellulose ;
   c) durcir ladite paroi externe en phtalate d'acétocellulose ;
   d) laver et sécher ladite aspirine enrobée d'une double paroi , et
   e) mélanger ladite aspirine enrobée d'une double paroi avec de l'aspirine non enrobée.

14. Le procédé selon la revendication 13, dans lequel ladite solution contenant du phtalate d'acétocellulose est tamponnée à un pH au-dessus de 5,5.

18

**15.** Le procédé selon la revendication 14, dans lequel ledit agent inducteur de séparation de phase est du sulfate de sodium aqueux dont la concentration est comprise entre 10 et 40 %.

**16.** Le procédé selon la revendication 15, dans lequel ledit durcisseur est de l'acide acétique.

**17.** Le procédé selon la revendication 16, dans lequel ledit pH est rabaissé au-dessous de 4 lors de l'addition dudit durcisseur.